(19) Europäisches Patentamt / European Patent Office / Office européen des brevets

(11) **EP 1 400 507 B1**

(12) **EUROPÄISCHE PATENTSCHRIFT**

(45) Veröffentlichungstag und Bekanntmachung des Hinweises auf die Patenterteilung:
**26.12.2012 Patentblatt 2012/52**

(51) Int Cl.:
***C07C 303/06*** *(2006.01)*   ***C07C 309/46*** *(2006.01)*

(21) Anmeldenummer: **03019620.8**

(22) Anmeldetag: **04.09.2003**

(54) **Verfahren zur Herstellung isolierter 3,4-Diaminobenzol-sulfonsäure**

PROCESS FOR THE PREPARATION OF ISOLATED 3,4-DIAMINOBENZENESULFONIC ACID

PROCEDE POUR LA PREPARATION D'ACIDE 3,4-DIAMINOBEZENESULFONIQUE ISOLE

(84) Benannte Vertragsstaaten:
**AT BE BG CH CY CZ DE DK EE ES FI FR GB GR HU IE IT LI LU MC NL PT RO SE SI SK TR**

(30) Priorität: **17.09.2002 DE 10243028**

(43) Veröffentlichungstag der Anmeldung:
**24.03.2004 Patentblatt 2004/13**

(73) Patentinhaber: **Symrise AG**
**37603 Holzminden (DE)**

(72) Erfinder:
• **Rauchschwalbe, Günter, Dr.**
 **51375 Leverkusen (DE)**
• **Emde, Herbert, Dr.**
 **51109 Köln (DE)**
• **Kissener, Wolfram, Dr.**
 **53819 Neunkirchen-Seelscheid (DE)**

(74) Vertreter: **Eisenführ, Speiser & Partner**
 **Postfach 10 60 78**
 **28060 Bremen (DE)**

(56) Entgegenhaltungen:
 **DE-A1- 2 131 367**

• **J.POST ET AL JUSTUS LIEBIGS ANN. CHEM. Nr. 205, 1880, Seiten 96 - 110, XP001091321**
• **PATENT ABSTRACTS OF JAPAN Bd. 006, Nr. 122, 7. Juli 1982 (1982-07-07) & JP 57 048961 A (SUMITOMO CHEM CO LTD), 20. März 1982 (1982-03-20)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gegenstand der Erfindung ist ein Verfahren zur Herstellung von 3,4-Diaminobenzolsulfonsäure durch Sulfieren von 1,2-Diaminobenzol mit Schwefelsäure, die kein oder nur bis 1,0 Mol $SO_3$ pro Mol enthält.

Ziel des erfindungsgemäßen Verfahrens ist ein einfaches Verfahren zur Herstellung von 3,4-Diaminobenzolsulfonsäure (auch "Orthaminsäure" genannten).

Diese Verbindung ist ein Zwischenprodukt zur Herstellung von Farbstoffen, Pflanzenschutzmittel, pharmazeutischen Produkten sowie von kosmetischen Produkten, besonders von UV-Absorptionsmitteln in Lichtschutzmitteln wie 2-Phenylbenzimidazol-monosulfonsäure. Besonders für die letztgenannten Verwendungszweck, wird besonders reine Ware benötigt, da die Endprodukte rein weiß sein sollen.

3,4-Diaminobenzolsulfonsäure kann nach früheren Befunden durch Umsetzung des HCl-Salzes von 1,2-Diaminobenzol mit einem großen Überschuß (ca. 11 mol/mol) $SO_3$-haltiger Schwefelsäure erhalten werden (siehe J. Post, Liebigs Ann. Chemie 205, (1880), S.100).

Die Verwendung des HCl-Salzes führt allerdings zur Entwicklung erheblicher Mengen an HCl-Gas, das korrosiv wirkt, die Verwendung geschlossener Apparate aus teuren Werkstoffen erfordert und die Wiederverwendung der Schwefelsäure behindern würde.

Es ist jedoch bisher nicht gelungen, sie aus einer Sulfierung von freiem 1,2-Diaminobenzol mit überschüssiger Schwefelsäure als isoliertes Produkt zu erhalten.

Nach dem Stand der Technik muss dazu der gesamte Überschuß an $H_2SO_4$ mit $BaCl_2$ ausgefällt und die restliche Mutterlauge eingedampft werden. Das so erhaltene Produkt kristallisiert schlecht und muss noch aufwendig gereinigt werden.

Häufig werden aromatische Sulfonsäuren zur Isolierung in Na-Salze überführt, die dann oft schwerer löslich sind als die reine Sulfosäure, doch ist dies am Beispiel der Orthaminsäure nicht bekannt.

Ein weiterer möglicher Weg zur Synthese der Orthaminsäure ist die Reduktion von 2-Nitro-anilin-4-sulfonsäure oder Dinitroazobenzoldisulfosäure mit Zinn/HCl (Zincke und Kuchenbecker, Ann.330, (1904), S. 23); doch sind diese Edukte nicht wohlfeil und müssen über mehrere Stufen synthetisiert werden.

Orthaminsäure wird dabei als HCl-Salz erhalten, das "sehr zersetzlich" ist (Nietzki, Lerch; Ber. d. deutsch. Chem. Ges. 21, 3220 (1888)). Erfahrungsgemäß wird die Orthaminsäure auf diesem Weg in unreiner, dunkel verfärbter Form erhalten, so dass eine Reinigung durch Umkristallisieren erforderlich ist (loc.cit.). HCl ist stark korrosiv; die Handhabung saurer, zersetzlicher HCl-Salze ist daher im technischen Maßstab nicht bevorzugt.

Die Synthese durch Sulfieren von 1,2-Diaminobenzol wäre dagegen vorteilhaft, da 1,2-Diaminobenzol in erheblichen Mengen erhältlich ist.

Erfahrungsgemäß ist es wünschenswert, Zwischenprodukte zu isolieren, wenn man sie in möglichst reiner Form erhalten und weiterverarbeiten will.

Außerdem ist es nicht immer möglich, rohe Reaktionslösungen ohne Zwischenisolierung weiterzuverarbeiten, die (außer Edukten und Folgeprodukten) wie in diesem Falle noch große Mengen Schwefelsäure enthalten können.

Das führt oft zur Bildung verunreinigter Produkte und/oder zu schlechten Ausbeuten in der Folgestufe.

Das ist zum Beispiel der Fall im Beispiel der EP-A 4203072; in diesem Falle wird eine Mischung aus 1,2-Diaminobenzol, Benzoesäure und 96%ige Schwefelsäure gemischt und bis auf 200°C erhitzt. Dabei wird das Folgeprodukt 2-Phenylbenzimidazol-5-Sulfonsäure in einer Ausbeute von 49 % d.Th. erhalten.

In einer weiteren Durchführung wird in 86 %iger Schwefelsäure bei 178° gearbeitet und das Produkt in einer Ausbeute von 60 % isoliert.

Die einzelnen Reaktionsschritte sind nicht beschrieben und daher unklar. Die Bildung oder gar Isolierung von Orthaminsäure auf diesem Wege ist nicht geschildert; das Verfahren, das mit wässriger Schwefelsäure und besonders hoher Temperatur arbeitet, legt das erfindungsgemäße Verfahren daher nicht nahe; der Anstieg der Ausbeute mit wachsendem Wassergehalt der Schwefelsäure in den geschilderten Beispielen lehrt geradezu von der Verwendung 100 %iger oder $SO_3$haltiger Schwefelsäure hinweg.

Das gilt dann ebenso für die Beispiele aus EP-A 1167358 und EP-A 1167359, die zeigen, dass bei dem Umsatz von Benzoesäure, 1,2-Diaminobenzol und Schwefelsäure in Anwesenheit erheblicher Mengen an $SO_3$ bei einer Verfahrenstemperatur von ca. 120° erhebliche Mengen oder gar ausschließlich die Disulfonsäuren des 2-Phenylbenzimidazols erhalten werden.

Eine Zwischenisolierung von Orthaminsäure, die durch Sulfieren erhalten wurde, ist heute auch aus ökonomischen wie ökologischen Gründen dringend gewünscht:

Da die Sulfierung in einem Überschuß von Schwefelsäure durchgeführt wird (siehe J.Post, loc. cit.), fällt dabei verunreinigte Schwefelsäure an, deren Wiederverwertung wünschenswert ist. Gebrauchte Schwefelsäure wird heute nach Stand der Technik unter Oxidation organischer Inhaltsstoffe entweder aufkonzentriert oder bei hoher Temperatur zu $SO_2$ zurückgespalten, um wieder in reine gebrauchsfähige Schwefelsäure überführt zu werden. Dazu muss selbstverständlich der gewünschte Wertstoff weitgehend vorher entfernt werden, und eine Verunreinigung mit anorganischen Ionen (wie bei der Fällung der Sulfonsäure als z.B. Na- oder K-Salz) ist untragbar, da das zu Störungen in der Aufarbeitung führen würde.

Außerdem wurde gefunden, dass bei der Sulfierung von 1,2-Diaminobenzol in Schwefelsäure, die eine überstöchiometrische Menge an $SO_3$ enthält, bereits erhebliche Mengen an Disulfonsäure entstehen.

Das Dokument JP57048961(A) offenbart ein Verfahren zur Herstellung von 2,4-Diaminobenzol-sulfonsäure durch Sulfonierung von meta-Diaminobenzol mit 65 Gew.-%iger rauchender Schwefelsäure, bei einer Temperatur von 120 bis 150°C. Zur Umwandlung von als Nebenprodukt entstandenem Disulfonierungsprodukt in das gewünschte Produkt, wird hierbei die Reaktionslösung nach der Reaktion mit Wasser verdünnt, bis eine Schwefelsäurekonzentration von 60 bis 90 Gew.-% erreicht wird und es wird nachfolgend auf 120 bis 150°C erhitzt.

Das Dokument DE 2 131 367 offenbart die Verwendung von 3,4-Diaminobenzol-sulfonsäure zur Herstellung von Benzimidazolon-5-sulfonsäure und die Herstellung von Produkten im Pharma-, Pflanzenschutz - oder Farbstoffbereich.

Es bestand daher die Aufgabe, einen Weg zu finden, Orthaminsäure durch Umsetzen von 1,2-Diaminobenzol mit Schwefelsäure herzustellen und in möglichst reiner Form, in hoher Ausbeute und frei von anorganischen Kationen daraus zu isolieren, so dass die eingesetzte Schwefelsäure wiederverwendet werden kann und Orthaminsäure in hoher Reinheit und Ausbeute daraus erhalten werden kann.

Es gelang nun überraschenderweise, Bedingungen zu finden, um 1,2-Diaminobenzol mit wasserfreier Schwefelsäure zu sulfieren, die kein $SO_3$ oder nur einen etwa molaren Anteil $SO_3$ enthält, und das Sulfiergemisch so aufzuarbeiten, dass 3,4-Diaminobenzol-sulfonsäure in hoher Ausbeute und hoher Reinheit erhalten wird, und dass die abgetrennte Schwefelsäure frei von anorganischen Kationen ist, die die Wiederverwertung beeinträchtigen würden.

Das ist besonders überraschend, da es unter sonst identischen Bedingungen nicht gelang, das strukturell sehr ähnliche 1,3-Diaminobenzol zu 1,3-Diaminobenzol-sulfonsäure so umzusetzen, dass sie aus dem Sulfiergemisch entsprechend isoliert werden könnte. In diesem Fall erreicht der Umsatz nur ca. 50 %.

Gegenstand der Erfindung ist daher ein Verfahren zur Herstellung von 3,4-Diaminobenzolsulfonsäure, dadurch gekennzeichnet, dass man

a) 1,2-Diaminobenzol mit wasserfreier Schwefelsäure, die gegebenenfalls $SO_3$ in bis zu stöchiometrischer Menge enthält, bei einer Temperatur im Bereich von 100 bis 160°C unter Rühren für eine Reaktionsdauer von 1 bis 20 Stunden umsetzt,

b) zu dem Reaktionsgemisch gegebenenfalls unter Kühlen Wasser oder Eis bis zu einer Konzentration der Schwefelsäure im Bereich von 30 bis 75 Gew.-%, bezogen auf das Gesamtgemisch, zugibt,

c) die aus dem Reaktionsgemisch ausgefallene 3,4-Diaminobenzolsulfonsärue abfiltriert, gegebenenfalls mit verdünnter Schwefelsäure wäscht und aufarbeitet.

Das Produkt, das nach dem genannten Verfahren erhalten wird, ist im Gegensatz zu früheren Befunden an Produkten anderer Herkunft nicht zersetzlich, sondern beständig.

Das Produkt, das nach diesem erfindungsgemäßen Verfahren erhalten wird, ist weiß bis dunkelgrau, i.a. hellgrau: Das ist besonders überraschend, da 1,2-Diaminobenzol leicht oxidabel ist und Schwefelsäure besonders bei erhöhter Temperatur als kräftiges Oxidationsmittel wirkt. Es war daher eher zu erwarten, dass die Sulfierung parallel mit Oxidation und der Bildung erheblicher Mengen dunkel gefärbter Nebenprodukte einhergehen würde, was zu schlechter Ausbeute hätte führen müssen.

Nach dem erfindungsgemäßen Verfahren wird 1,2-Diaminobenzol in einen Überschuß von 100%iger Schwefelsäure bzw. einen Überschuß an $SO_3$-haltiger Schwefelsäure (aber in einer Menge an $SO_3$, die unter 1 mol pro mol Phenylendiamin liegt) eingebracht, einige Stunden erhitzt, ggf. mit weiterer Schwefelsäure verdünnt und dann mit soviel Wasser verdünnt, dass eine Schwefelsäure bestimmter Konzentration entsteht; dabei fällt Orthaminsäure in hoher Ausbeute in kristalliner Form aus und kann durch geeignete Maßnahmen wie Filtrieren, oder Zentrifugieren abgetrennt werden.

Eine Wäsche mit Schwefelsäure bestimmter Verdünnung ist möglich, in der Regel aber nicht erforderlich, da das Produkt bereits in hoher Reinheit anfällt (isomerenrein laut 1H-NMR-Spektrum). Das ist um so überraschender, als im elektronenreichen aromatischen Kern des 1,2-Diaminobenzols 2 in ihrer Reaktivität sehr ähnliche Positionen zur Verfügung stehen und daher mit der Bildung vergleichbarer Mengen an 3,4- sowie 2,3-Diaminobenzol-sulfonsäure gerechnet werden musste. Nichtsdestoweniger beträgt die Ausbeute an isolierter Ware bis 92-94 % d.Th. an Orthaminsäure, bezogen auf eingesetztes 1,2-Diaminobenzol (Bestimmung durch HPLC oder Nitrit-Titration), die laut 1H-NMR-Spektrum isomerenfrei erhalten wird.

Je nach Reaktionsbedingungen kann als Nebenprodukt (in einer Menge von wenigen %) 1,2-Diaminobenzoldisulfonsäure entstehen; diese wird bei der Aufarbeitung abgetrennt und ist im Produkt praktisch nicht enthalten (Gehalt <1,0%).

Je nach Reaktionsbedingungen kann auch noch Edukt in geringer Menge unumgesetzt zurückbleiben; dieses wird bei der Aufarbeitung abgetrennt und ist im Produkt nicht mehr enthalten.

Wird bei Verwendung von etwa stöchiometrischer Menge an $SO_3$ vermehrt Disulfonsäure gebildet, so kann durch Zusatz von Wasser nach der Sulfierung (aber vor der Aufarbeitung) ihr Anteil stark zurückgeführt werden.

Die Sulfierung erfolgt in einem Temperaturbereich von 90 bis 160°C.

In einer bevorzugten Durchführungsform wird 1,2-Diaminobenzol in 3-5fachen Gewichtsmenge 100 %iger Schwefelsäure gelöst. (Die genaue Menge wird so festgelegt, dass der Ansatz in der Aufarbeitung noch gut gerührt bzw. gefördert werden kann.)

Die Lösung wird auf eine Temperatur von 100°C bis 160°C erhitzt; In einer besonderen bevorzugten Durchführungsform auf eine Temperatur von 120-150° erhitzt und 1 bis 20 Stunden, insbesondere 4-15 Stunden, bevorzugt 6-10 Stunden gerührt.

In einer besonderen Durchführungsform wird nach Durchführung der Sulfierreaktion vor dem Verdünnen mit viel Wasser zunächst wenig Wasser zugegeben und der Ansatz noch 1-3 Stunden bei der Verfahrenstemperatur nachgerührt, wobei der ohnehin sehr geringe Gehalt an Disulfonsäure weiter verringert werden kann.

Danach wird ggf. nach Abkühlen auf 100°C auf Wasser oder Eis ausgetragen, so dass die Konzentration der Schwefelsäure nach der Verdünnung 30 bis 75 % beträgt, bevorzugt ist eine Konzentration von 40 bis 70 %, ganz besonders bevorzugt ist eine Konzentration von 50 bis 65%, da das Produkt dann besonders gute Filtrationseigenschaften aufweist und besonders wenig löslich ist.

Wird beim Verdünnen der Schwefelsäure die Vorlage nicht gekühlt, so bleibt dabei das Produkt zunächst in Lösung, da die Vorlage sich durch die Verdünnungswärme erhitzt. Beim Kaltrühren kristallisiert dann das Produkt aus.

In einer besonderen Durchführungsform wird Schwefelsäure der gewünschten Verdünnung vorgelegt und bei der gewünschten Temperatur sowohl Wasser als auch der Sulfieransatz simultan so eindosiert, dass die Konzentration der Schwefelsäure (berechnet aus Schwefelsäure und Wasser) in der Vorlage konstant bleibt.

Dabei ist es wünschenswert, dass die Temperatur in der Vorlage so niedrig gehalten wird, dass das Produkt bereits während der Dosierung auskristallisiert.

Dann wird das ausgefallene Produkt abfiltriert. Es wird auch ohne Waschen in hinreichend reiner Form erhalten, kann aber durch Nachwaschen mit verdünnter Schwefelsäure noch weiter gereinigt werden. Das Produkt enthält dann noch wenig anhaftende Schwefelsäure, die aber in der Regel für die Weiterverarbeitung nicht weiter entfernt werden muss.

Der Gehalt an gewünschtem Produkt kann auf einfache Weise bestimmt werden, z.B. durch HPLC oder Diazotierung. Er beträgt in der Regel 30-50 %, meistens 45-50 %, je nach Aufarbeitungsbedingungen.

Die ablaufende Dünnsäure enthält nur wenig organisches Material (<ca. 1 Gew.-% Orthaminsäure sowie kleine Mengen Disulfonsäure), so dass sie ggf. nach einer Aufarbeitung wiederverwendet werden kann.

Das isolierte Produkt ist lt. 1H-NMR-Spektroskopie (400 MHz) frei von Isomeren:

$$(DMSO\text{-}d6)\ \delta = 6{,}92\ (d, J = 8{,}3Hz),\ 7{,}24\ (d/d, J = 1{,}92/8{,}3Hz),\ 7{,}43\ (d, J = 1{,}91Hz),$$

$$8{,}1\ s(breit).$$

So erhaltene Rohware ist daher bereits für viele Zwecke brauchbar, zum Beispiel zur Herstellung von Produkten der pharmazeutischen oder der Agrochemie, für Farbstoffe oder kosmetische Produkte. Die hergestellten 3,4-Diaminobenzolsulfonsäure können zur Herstellung von Produkten in Pharma-, Pflanzenschutz-, Kosmetikoder Farbstoffbereich verwendet werden.

Optional kann das Produkt auch aus z.B. der drei- bis fünffachen Gewichtsmenge Wasser umkristallisiert werden, um besonders reine, z.B. von $H_2SO_4$ freie Ware zu erhalten.

So umkristallisierte Ware ergibt korrekte Elementaranalysen:

| Ber. C: | 38,3% | gef.C: | 38,0% |
|---------|-------|--------|-------|
| Ber.H: | 4,3% | gef.H: | 4,1% |
| Ber.N: | 14,88% | gef.N: | 14,8% |
| Ber.S: | 17,03% | gef.S: | 17,4% |

Gehalt aus Diazotierung: 99,6%

Infrarotspektrum:

v = 3454(w), 3364(w), 2873(s,br), 2638(s), 1639(s), 1556(m), 1502(s), 1316(w) 1230(s), 1207(s), 1168(m), 1145 (s), 1106(s), 1021(s), 819(w). (s: strong, m: medium, w: weak),

Die 3,4-Diaminobenzolsulfonsäure kann durch Lösen in heißem Wasser und Aktivkohlebehandlung aufgearbeitet werden.

**Beispiel 1**

Man legt 600 ml (1110 g) 100 %ige Schwefelsäure vor, löst darin 216 g (2,0 Mol) 1,2-Diaminobenzol und erhitzt 2-3 Stunden auf 100°C.

Dann tropft man 275 g 65 % $SO_3$ enthaltende Schwefelsäure zu (2,23 Mol $SO_3$) und rührt noch 2 Stunden bei 140°C.

Man kühlt auf 125°C ab und tropft 70 ml Wasser zu. Man rührt 2 weitere Stunden bei 135-140°C, kühlt auf 100°C ab und trägt unter Rühren innerhalb von 15 Minuten auf 1300 ml Wasser aus.

Man rührt innerhalb von 6 Stunden kalt bis auf 25°C und filtriert über ein säurefestes Filter.

Man erhält 724 g 47,6 %ige Orthaminsäure (91,5 % d.Th.) und 2060 g (1,6 l) salzfreie Dünnsäure, die noch 0,25 % Diaminobenzol sowie 0,6 % Orthaminsäure enthält.

Das graue Produkt kann ohne weitere Reinigung verwendet werden.

**Beispiel 2**

Man legt 368 g (3,76 mol) 100 %ige Schwefelsäure vor, löst darin 48,5 g (0,257 mol) 1,2-Diaminobenzol und erhitzt auf 135°C, später auf 138°C:

Man verfolgt die Reaktion durch HPLC (Angaben in rel. Mol-%)

| Reaktionsbedingungen | Edukt | Produkt (Monosulfonsäure) | Folgeprodukt (Disulfonsäure) |
|---|---|---|---|
| 6h/135° | 7,2 | 90,95 | 1,9 |
| 9h/135° | 6,1 | 92,05 | 1,8 |
| +3h/138° | 5,7 | 92,37 | 1,9 |
| +3h/138° | 3,6 | 94,50 | 1,95 |

Man trägt auf 200g Wasser aus und isoliert Orthaminsäure, die in kristalliner Form erhalten wird (Beurteilung unter dem Mikroskop bei 10facher Vergrößerung; Ausbeute 96 % d.Th.).

**Beispiel 3**

Man verfährt wie in Beispiel 1, erhitzt jedoch 8h auf 138°C.

Man erhält Orthaminsäure in einer Ausbeute von 93,8 % d. Th..

| Reaktions-bedingungen | Edukt | Produkt | Folgeprodukt |
|---|---|---|---|
| 8h/138° | 2,8 | 95,4 | 1,7 |
| Isoliertes Produkt | 0,6 | 98,8 | 0,6 |

**Beispiel 4**

Man legt 313 g (3,19 mol) Schwefelsäure vor, löst darin 48,5 g (0,257 mol) 1,2-Diaminobenzol und erhitzt auf 138°C.

Später gibt man noch 10 g Schwefelsäure zu, die 65% $SO_3$ (0,08 mol) enthält.

Man verfolgt die Reaktion durch HPLC (Angaben in rel. mol-%).

Man trägt auf 200 ml Wasser aus kühlt auf 40° ab und isoliert das ausgefallene Produkt durch Filtration. Man erhält Orthaminsäure in einer Ausbeute von 98,1% d. Th.

| Reaktions-bedingungen | Edukt | Produkt (Orthaminsäure) | Folgeprodukt (Disulfonsäure) |
|---|---|---|---|
| 6 h / 138° | 4,1 | 91,2 | 2,1 |
| Nachsatz Oleum und 3h/138° | 3,1 | 91,9 | 4,0 |
| Isoliertes Produkt | 0,4 | 98,0 | 1,6 |

**Beispiel 5**

Man legt 313 g Schwefelsäure vor, löst darin 48,5 g 1,2-Diaminobenzol und erhitzt 8h auf 145°C.
Man verfolgt die Ergebnisse durch HPLC.
Man trägt auf 220 ml Wasser aus, kühlt ab und isoliert durch Filtration.
Man isoliert Orthaminsäure in einer Ausbeute von 93,8 % d.Th.

| Reaktions bedingurigen | Edukt | Produkt (Orthaminsäure) | Folgeprodukt (Disulfonsäure) |
|---|---|---|---|
| 8h/145° | 1,9 | 94,3 | 3,8 |
| Isoliertes Produkt | 1,1 | 98,3 | 0,6 |

**Beispiel 6**

Man löst 970 g 1,2-Diaminobenzol (8,965 mol) in 3786 ml (7000 g /71,5 mol; 8 mol/mol) 100%iger Schwefelsäure ("Monohydrat") und erhitzt 8 Stunden auf 138°C.
Man kühlt auf 100°C ab, verdünnt mit 925 g Schwefelsäure und trägt auf 5,05 1 Wasser aus.
Man kühlt auf 40°C ab und erhält Orthaminsäure mit einem durchschnittlichen Gehalt von ca. 50 % (bestimmt durch Diazotieren und Korrektur durch HPLC) in einer Ausbeute von ca. 94 % d.Th.

**Beispiel 7**

600 g schwefelsäurefeuchte rohe Orthaminsäure wird in 1700 ml Wasser heiß gelöst, mit 8 g Aktivkohle 30 Minuten bei 80° gerührt, heiß filtriert und kalt gerührt.
Das ausgefallene Produkt wird abgesaugt und getrocknet.
Man erhält 135 g Trockenware, die laut HPLC einen Gehalt von 98,8 % aufweist.
Die ablaufende Dünnsäure enthält 0,9 % Orthaminsäure.

**Beispiel 8**

Man löst 48,7 g 1,2-Diaminobenzol in 249 g/2,53 mol (5,7 mol/mol) 100 %iger Schwefelsäure und erhitzt. Man verfolgt die Ergebnisse durch HPLC.

| Reaktionsbedingungen: Temperatur | Edukt | Produkt (Orthaminsäure) |
|---|---|---|
| 1h /80° * | 96,2 | 2,4 |
| 2h180° * | 96,1 | 3,3 |
| 2 h /90° * | 88,9 | 7,1 |
| 2 h /100° | 83,6 | 12,1 |
| 2 h /110° | 71,6 | 24,8 |
| 2 h /120° | 51,3 | 41,7 |
| 2 h /130° | 15,0 | 84,0 |

(fortgesetzt)

| Reaktionsbedingungen: Temperatur | Edukt | Produkt (Orthaminsäure) |
|---|---|---|
| 2h/140° | 5,5 | 93,3 |

* nicht erfindungsgemäß; die Angaben dienen dazu, das Verständnis der Erfindung zu erleichtern.

**Beispiel 9**

Man löst 49 g 1,2-Diaminobenzol in 276 g (2,82 mol; 6,2 mol/mol) und erhitzt auf 135°C.

Man verfolgt die Reaktionsergebnisse durch HPLC.

| Reaktionsbedingungen: Zeit [h] | Edukt | Produkt (Orthaminsäure) | Folgeprodukt (Disulfonsäure) |
|---|---|---|---|
| 2 | 27,2 | 69,2 | 3.8 |
| 4 | 14,0 | 81,9 | 4,2 |
| 5 | 10,5 | 86,7 | 2,8 |
| 6 | 8,3 | 88,8 | 2,9 |
| 8 | 6,6 | 90,6 | 2,8 |
| 10 | 5,3 | 92,2 | 2,6 |
| 12 | 6,4 | 91,1 | 2,5 |
| 14 | 5,3 | 92,7 | 2,3 |
| 16 | 4,9 | 93,9 | 2,3 |

**Vergleichsbeispiel:**

48,5 g 1,3-Diaminobenzol wurden in 170 ml 100 %iger Schwefelsäure gelöst und langsam erhitzt. Der Umsatz wurde durch HPLC verfolgt. Der Umsatzgrad erreichte bei 140° auch nach 13 h nur maximal 48 mol-%.

Erhöhung der Temperatur auf 160°C erhöhte den Umsatz nach 2 weiteren Stunden nur auf 51 %.

**Patentansprüche**

**1.** Verfahren zur Herstellung von 3,4-Diaminobenzolsulfonsäure, **dadurch gekennzeichnet, dass** man

a) 1,2-Diaminobenzol mit wasserfreier Schwefelsäure, die gegebenenfalls $SO_3$ in bis zu stöchiometrischer Menge enthält, bei einer Temperatur im Bereich von 100 bis 160 °C unter Rühren für eine Reaktionsdauer von 1 bis 20 Stunden umsetzt,

b) zu dem Reaktionsgemisch gegebenenfalls unter Kühlen Wasser oder Eis bis zu einer Konzentration der Schwefelsäure im Bereich von 30 bis 75 Gew.%, bezogen auf das Gesamtgemisch, zugibt,

c) die aus dem Reaktionsgemisch ausgefallene 3,4-Diaminobenzolsulfonsäure abfiltriert, gegebenenfalls mit verdünnter Schwefelsäure wäscht und aufarbeitet.

**2.** Verfahren nach Anspruch 1, **dadurch gekennzeichnet, dass** die 3,4-Diaminobenzolsulfonsäure aus Wasser umkristallisiert wird.

**3.** Verfahren nach Anspruch 1, **dadurch gekennzeichnet, dass** man die 3,4-Diaminobenzolsulfonsäure durch Lösen in heißem Wasser und Aktivkohlebehandlung aufarbeitet.

**4.** Verfahren nach einem der Ansprüche 1-3, **dadurch gekennzeichnet, dass** die Schwefelsäure bzw. Schwefelsäureäquivalent in einem molaren Verhältnis von 5 bis 15 zu 1 eingesetzt wird und dass dabei $SO_3$ in einem molaren Verhältnis von 1,5 bis 0 zu 1 eingesetzt wird.

**5.** Verfahren nach einem der Ansprüche 1 bis 4, **dadurch gekennzeichnet, dass** die Reaktion in Schritt a) in einem Temperaturbereich von 120°C bis 150 °C durchgeführt wird.

**6.** Verfahren nach Anspruch 1, **dadurch gekennzeichnet, dass** die Reaktion in Schritt a) in einer Reaktionszeit von 5 bis 15 Stunden durchgeführt wird.

**7.** Verfahren nach Anspruch 1, **dadurch gekennzeichnet, dass** die Dosierung der Reaktionslösung und des verdünnenden Wassers in Schritt b) simultan so erfolgt, dass die Schwefelsäurekonzentration in der Vorlage nur um +/- 5% schwankt.

**Claims**

**1.** Process for the preparation of 3,4-diaminobenzenesulphonic acid, **characterised in that**

a) 1,2-diamonobenzene is converted with anhydrous sulphuric acid, which optionally contains $SO_3$ in an up to stoichiometric amount, at a temperature in the range from 100° to 160°C while stirring for a reaction time of 1 to 20 hours,
b) water or Ice is added to the reaction mixture, optionally while cooling, until the concentration of sulphuric acid is in the range from 30 to 75 wt.%, referred to the total mixture,
c) the 3,4-diaminobenzenesulphonic acid precipitated from the reaction mixture is filtered off, optionally washed with dilute sulphuric acid, and worked up.

**2.** Process according to claim 1, **characterised in that** the 3,4-diaminobenzenesulphonic acid is recrystallised from water.

**3.** Process according to claim 1, **characterised in that** the 3,4-diaminobenzenesulphonic acid is worked up by dissolving it in hot water and treatment with activated charcoal.

**4.** Process according to one of claims 1 to 3, **characterised in that** the sulphuric acid or sulphuric acid equivalent is used in a molar ratio of 5 to 15 : 1, and $SO_3$ is used in a molar ratio of 1.5 to 0 : 1.

**5.** Process according to one of claims 1 to 4, **characterised in that** the reaction in step a) is carried out in a temperature range from 120° to 150°C.

**6.** Process according to claim 1, **characterised in that** the reaction in step a) is carried out in a reaction time of 5 to 15 hours.

**7.** Process according to claim 1, **characterised in that** the metering in of the reaction solution and of the diluting water in step b) takes place simultaneously so that the sulphuric acid concentration in the receiver varies only by ± 5%.

**Revendications**

**1.** Procédé de production d'acide 3,4-diaminobenzène-sulfonique, **caractérisé en ce que**

a) l'on fait réagir du 1,2-diaminobenzène avec de l'acide sulfurique anhydre qui contient éventuellement du $SO_3$, jusqu'à une quantité stoechiométrique, à une température dans une plage de 100 à 160° C sous agitation pendant une durée de réaction comprise entre 1 et 20 heures,
b) au mélange réactionnel, on ajoute éventuellement en refroidissant, de l'eau ou de la glace jusqu'à une concentration d'acide sulfurique dans une plage de 30 à 75 % en masse par rapport au mélange total,
c) on filtre l'acide 3,4-diaminobenzène-sulfonique précipité du mélange réactionnel, éventuellement on le lave et on le traite avec de l'acide sulfurique dilué.

**2.** Procédé selon la revendication 1, **caractérisé en ce que** l'acide 3,4-diaminobenzène-sulfonique est recristallisé à partir de l'eau.

**3.** Procédé selon la revendication 1, **caractérisé en ce que** l'on traite l'acide 3,4-diaminobenzène-sulfonique par dissolution dans de l'eau chaude et traitement par charbon actif.

4. Procédé selon l'une des revendications 1 à 3, **caractérisé en ce que** l'acide sulfurique ou un équivalent d'acide sulfurique est utilisé en un rapport molaire de 5 à 15 pour 1 et **en ce que** le $SO_3$ est alors utilisé en un rapport molaire de 1,5 à 0 pour 1.

5. Procédé selon l'une des revendications 1 à 4, **caractérisé en ce que** la réaction à l'étape a) est réalisée dans une plage de températures de 120° C à 150° C.

6. Procédé selon la revendication 1, **caractérisé en ce que** la réaction à l'étape a) est réalisée en un temps de réaction compris entre 5 et 15 heures.

7. Procédé selon la revendication 1, **caractérisé en ce que** le dosage de la solution réactionnelle et de l'eau diluée à l'étape b) s'effectue simultanément de telle sorte que la concentration d'acide sulfurique ne varie dans le récipient que de +/- 5 %.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 4203072 A
- EP 1167358 A
- EP 1167359 A
- JP 57048961 A
- DE 2131367

### In der Beschreibung aufgeführte Nicht-Patentliteratur

- *J. Post, Liebigs Ann. Chemie,* 1900, vol. 205, 100
- **ZINCKE ; KUCHENBECKER.** *Ann.,* 1904, vol. 330, 23